# EUROPEAN PATENT SPECIFICATION

(11) **EP 3 919 965 B1**
(45) Date of publication and mention of the grant of the patent: **01.01.2025**
(21) Application number: 20762127.7
(22) Date of filing: 07.02.2020
(51) Int. Cl.: G02C 7/04, G02C 11/00, G02C 13/00, A61F 9/00

(54) **CONTACT LENS AND PAIR OF CONTACT LENSES**
KONTAKTLINSE UND PAAR VON KONTAKTLINSEN
LENTILLE DE CONTACT ET PAIRE DE LENTILLES DE CONTACT

(30) Priority: 28.02.2019 JP 2019035150
(43) Date of publication of application: 08.12.2021
(73) Proprietor: Sony Group Corporation, Tokyo 108-0075 (JP); Sony Semiconductor Solutions Corporation, Atsugi-shi, Kanagawa 243-0014 (JP)
(72) Inventor: IWASAKI, Masanori, Tokyo 108-0075 (JP); YOSHIMURA, Tsukasa, Tokyo 141-0022 (JP); HIRABAYASHI, Takayuki, Atsugi-shi, Kanagawa 243-0014 (JP); HAYAKAWA, Ken, Tokyo 108-0075 (JP); SHIGA, Fumiko, Tokyo 108-0075 (JP)
(74) Representative: D Young & Co LLP
(86) International application number: PCT/JP2020/004801
(87) International publication number: WO 2020/175104

(56) References cited:
- FR-A1- 2 443 233
- JP-A- 2006 230 799
- JP-A- 2017 517 762
- JP-A- 2020 003 586
- JP-A- H01 230 341
- JP-A- H05 293 090
- KR-B1- 101 860 030
- US-A- 4 109 648
- US-A1- 2014 379 054
- US-A1- 2014 379 054

## Description

### Technical Field

The present disclosure relates to a contact lens, a pair of contact lenses, and a wearing device.

### Background Art

In recent years, a method has been developed that acquires biological information and the like with use of a contact lens.

### Citation List

### Patent Literature

PTL 1: Japanese Unexamined Patent Application Publication (Published Japanese Translation of PCT Application) No. 2017-523482
PTL 2: Japanese Unexamined Patent Application Publication (Published Japanese Translation of PCT Application) No. 2013-544558
PTL 3: Japanese Unexamined Patent Application Publication (Published Japanese Translation of PCT Application) No. 2013-518672
PTL 4: Japanese Unexamined Patent Application Publication (Published Japanese Translation of PCT Application) No. 2004-530504
PTL 5: Japanese Unexamined Patent Application Publication (Published Japanese Translation of PCT Application) No. 2016-515897
Cited references also include US 2014379054 A1, KR 101860030 B1, FR 2443233 A1, JP 2006230799 A and US 4109648 A.

### Summary of the Invention

Incidentally, in a case where data is transmitted from a contact lens to peripheral equipment, wireless communication is typically used. In a case where the contact lens is worn on an eyeball, the area of the contact lens is small, and the contact lens gets wet with tears, which easily causes a decrease in communication sensitivity, and easily causes a slowdown in communication speed by noise superimposed on a signal. In addition, it is difficult to mount a battery having a large capacity on the contact lens, which limits functions implementable in the contact lens, and long-time use of a battery is not expected. It is possible to wirelessly supply electric power to the contact lens. However, it is difficult to dispose an electric power source in proximity to the contact lens, which limits suppliable electric power. As described above, in wireless communication, limitations are put on a communication band, a mounting function, used electric power, supplied electric power, a driving time, and the like. In addition, the contact lens is physically isolated from outside except for an eye; therefore, it is not possible to exchange an object between the contact lens and the outside. As described above, due to various limitations described above, it has been extremely difficult to provide a contact lens as a wearable device that is endurable for practical use. It is therefore desirable to provide a contact lens and a pair of contact lenses that make it possible to reduce limitations on being practically used as a wearable device.

The invention is defined by the claims.

### Brief Description of Drawings

[FIG. 1] FIG. 1 is a diagram illustrating an example of a state in which a contact lens according to a first embodiment of the present disclosure is worn on an eyeball.
[FIG. 2] FIG. 2 is a diagram illustrating a modification example of the contact lens in FIG. 1.
[FIG. 3] FIG. 3 is a diagram illustrating an example of a line, which is physically coupled to the contact lens, of an external device.
[FIG. 4] FIG. 4 is a diagram illustrating an example of a state in which contact lenses are worn on both eyes.
[FIG. 5] FIG. 5 is a diagram illustrating an example of a cross-sectional configuration of the line, which is physically coupled to the contact lens, of the external device.
[FIG. 6] FIG. 6 is a diagram illustrating a modification example of a state in which the contact lenses are worn on both eyes.
[FIG. 7] FIG. 7 is a diagram illustrating a state in which a plurality of terminals is provided in the contact lens.
[FIG. 8] FIG. 8 is a diagram illustrating an example of a state in which the contact lens in FIG. 7 and the external device are physically coupled to each other by a plurality of lines of the external device.
[FIG. 9] FIG. 9 is a diagram illustrating a modification example of the cross-sectional configuration of the line, which is physically coupled to the contact lens, of the external device.
[FIG. 10] FIG. 10 is a diagram illustrating a modification example of the cross-sectional configuration of the line, which is physically coupled to the contact lens, of the external device.
[FIG. 11] FIG. 11 is a diagram illustrating a modification example of the cross-sectional configuration of the line, which is physically coupled to the contact lens, of the external device.
[FIG. 12] FIG. 12 is a diagram illustrating an example of a state in which a contact lens according to a second embodiment of the present disclosure is worn on an eyeball.
[FIG. 13] FIG. 13 is a diagram illustrating an example of placement of a line, which is physically coupled to an external device, of the contact lens.
[FIG. 14] FIG. 14 is a diagram illustrating an example of a state in which contact lenses are worn on both eyes.
[FIG. 15] FIG. 15 is a diagram illustrating an example of a cross-sectional configuration of a line, which is physically coupled to the external device, of the contact lens.
[FIG. 16] FIG. 16 is a diagram illustrating a modification example of the state in which the contact lenses are worn on both eyes.
[FIG. 17] FIG. 17 is a diagram illustrating a state in which a plurality of terminals is provided in the contact lens.
[FIG. 18] FIG. 18 is a diagram illustrating an example of a state in which the contact lens in FIG. 17 and the external device are physically coupled to each other by a plurality of lines of the contact lens.
[FIG. 19] FIG. 19 is a diagram illustrating a modification example of a cross-sectional configuration of a line, which is physically coupled to the external device, of the contact lens.
[FIG. 20] FIG. 20 is a front view of an example of a state in which a light guiding section is provided in the contact lens in FIG. 12.
[FIG. 21] FIG. 21 is a diagram illustrating an example of a cross-sectional configuration of the light guiding section in FIG. 20.
[FIG. 22] FIG. 22 is a front view of an example of a state in which in a light guiding section and a light control section are provided in the contact lens in FIG. 12.
[FIG. 23] FIG. 23 is a diagram illustrating an example of a cross-sectional configuration of the light guiding section in FIG. 22.
[FIG. 24] FIG. 24 is a diagram illustrating an example of a cross-sectional configuration of a line, which is physically coupled to the external device, of the contact lens.
[FIG. 25] FIG. 25 is a diagram illustrating an example of a cross-sectional configuration of a line, which is physically coupled to the external device, of the contact lens.
[FIG. 26] FIG. 26 is a diagram illustrating a modification example of placement of the line, which is physically coupled to the external device, of the contact lens.
[FIG. 27] FIG. 27 is a diagram illustrating a modification example of placement of the line, which is physically coupled to the external device, of the contact lens.
[FIG. 28] FIG. 28 is a diagram illustrating an embodiment of the invention.
[FIG. 29] FIG. 29 is a front view of an example of a wearing device for a contact lens.
[FIG. 30] FIG. 30 is a diagram illustrating a back configuration example of the wearing device in FIG. 29.
[FIG. 31] FIG. 31 is a diagram illustrating an example of a cross-sectional configuration taken along a line A-A of FIG. 30.
[FIG. 32] FIG. 32 is a diagram illustrating a state in which a contact lens is placed on the wearing device in FIG. 29.
[FIG. 33] FIG. 33 is a diagram illustrating a modification example of a back configuration of the wearing device in FIG. 29.
[FIG. 34] FIG. 34 is a diagram illustrating an example of a state in which an antenna line is provided in an external device coupled to the contact lens according to any of the second embodiment and modification examples thereof.
[FIG. 35] FIG. 35 is a diagram illustrating a modification example of the external device in FIG. 34.
[FIG. 36] FIG. 36 is a diagram illustrating a modification example of the external device in FIG. 34.
[FIG. 37] FIG. 37 is a diagram illustrating a state in which one antenna line is provided in the contact lens in FIG. 17.
[FIG. 38] FIG. 38 is a diagram illustrating a state in which two antenna lines are provided in the contact lens in FIG. 17.
[FIG. 39] FIG. 39 is a diagram illustrating an example of a state in which an antenna line is provided in an external device coupled to the contact lens according to any of the first embodiment and modification examples thereof.
[FIG. 40] FIG. 40 is a diagram illustrating a modification example of the external device in FIG. 39.
[FIG. 41] FIG. 41 is a diagram illustrating a modification example of the external device in FIG. 39.
[FIG. 42] FIG. 42 is a diagram illustrating a modification example of the external device in FIG. 39.
[FIG. 43] FIG. 43 is a diagram illustrating a modification example of the external device in FIG. 39.
[FIG. 44] FIG. 44 is a diagram illustrating an example of a contact lens coupled to the external device in FIG. 43.
[FIG. 45] FIG. 45 is a diagram illustrating a modification example of the external device in FIG. 39.
[FIG. 46] FIG. 46 is a diagram illustrating an example of a contact lens coupled to the external device in FIG. 45.
[FIG. 47] FIG. 47 is a diagram illustrating a modification example of the contact lens.

### Modes for Carrying Out the Invention

Some embodiments of the present disclosure are described below in detail with reference to the drawings. It is to be noted that the description is given in the following order.

### 1. First Embodiment

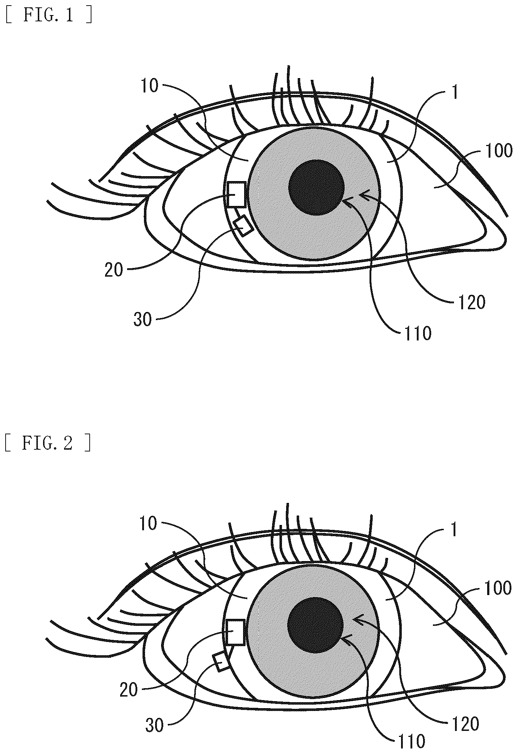

An example in which a terminal is provided in a lens section (FIGs. 1 to 5)

### 2. Modification Examples of First Embodiment

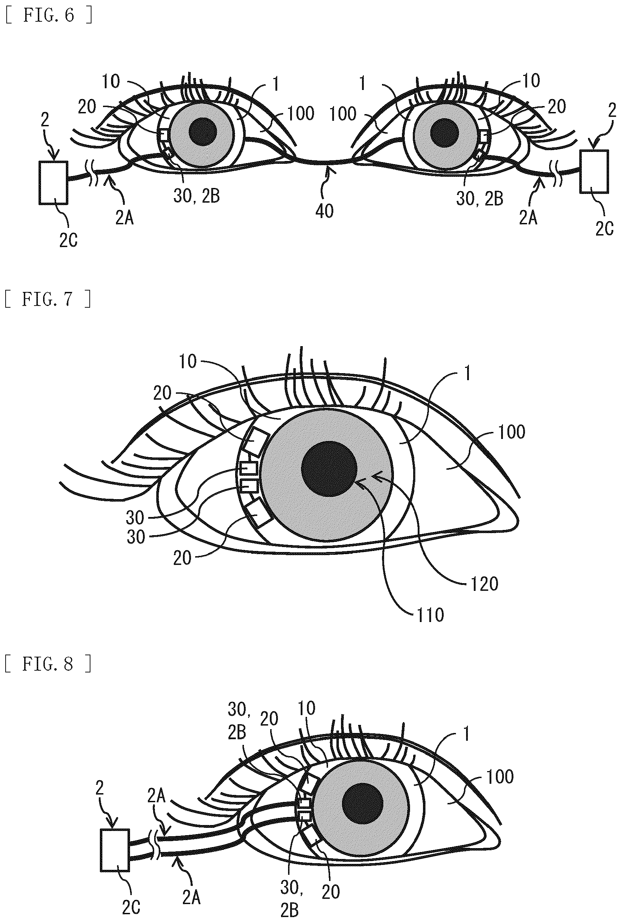

Variations of a line that couples a contact lens and an external device to each other (FIGs. 6 to 11)

### 3. Second Embodiment

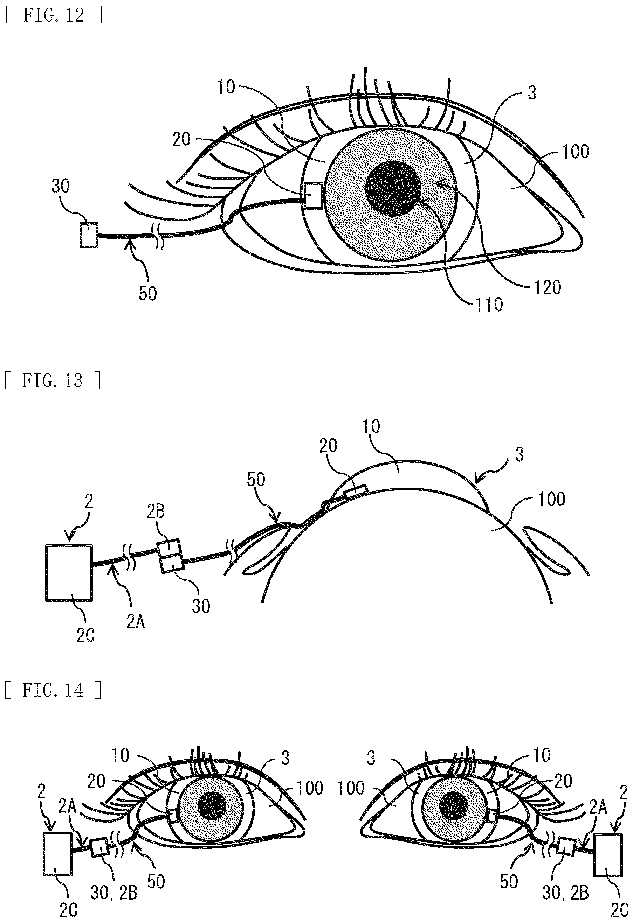

An example in which a lens section and a terminal are coupled to each other by a line (FIGs. 12 to 15)

### 4. Modification Examples of Second Embodiment

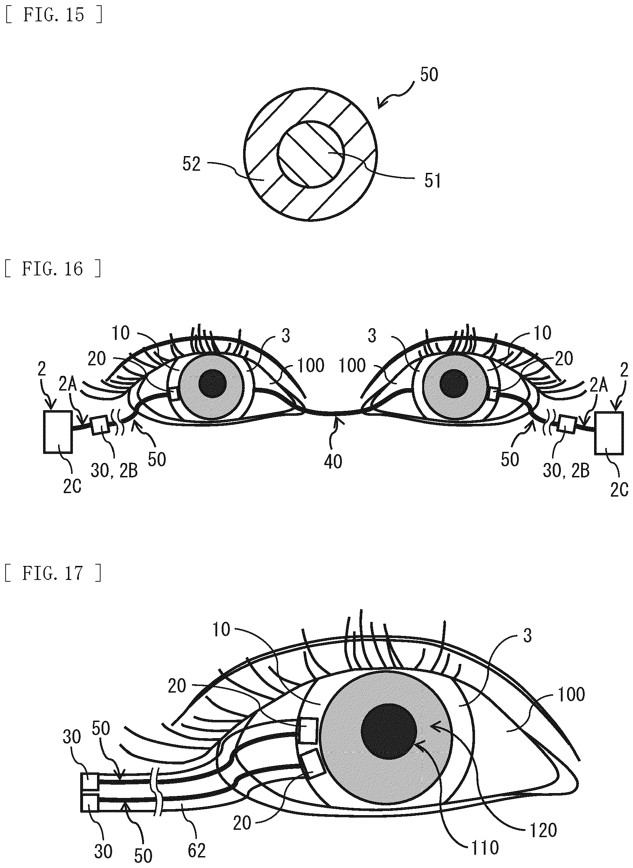

Variations of the line that couples the lens section and the terminal to each other (FIGs. 16 to 28)

### 5. Third Embodiment

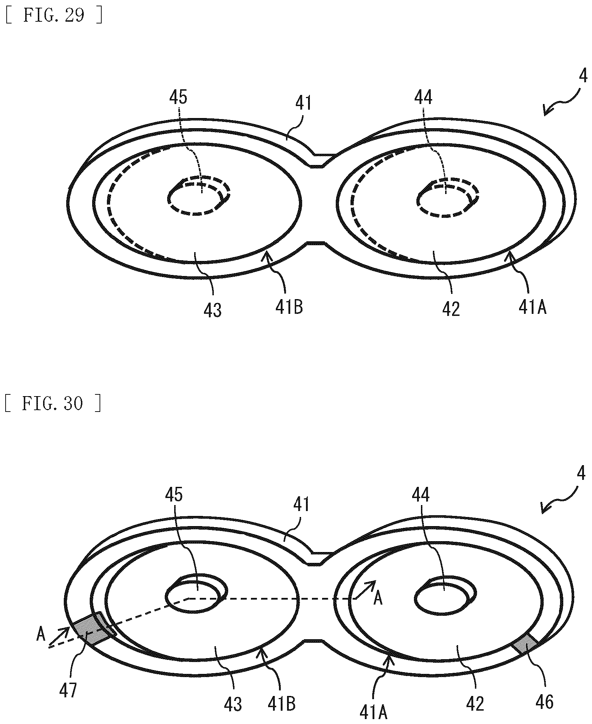

A wearing device for a contact lens (FIGs. 29 to 33)

### 6. Common Modification Examples for First and Second Embodiments

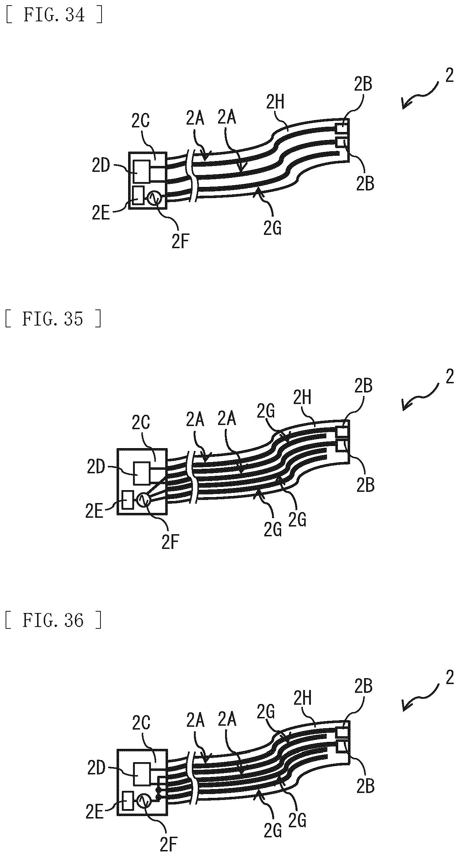

Examples in which an antenna is provided in an external device or a contact lens (FIGs. 34 to 47)

### < 1.First Embodiment>

### [Configuration]

Description is given of a contact lens 1 according to a first embodiment of the present disclosure. FIG. 1 illustrates an example of a state in which the contact lens 1 is worn on an eyeball 100. The contact lens 1 performs wired communication with an external device 2 to be described later. The eyeball 100 includes a pupil 110 and an iris 120. An upper portion and a lower portion of the eyeball 100 are covered with an upper eyelid and a lower eyelid, and an upper portion and a lower portion of the contact lens 1 worn on the eyeball 100 are also covered with the upper eyelid and the lower eyelid.

The contact lens 1 includes a lens section 10 worn on the eyeball 100, a functional section 20 provided in the lens section 10, and a terminal 30 that is physically coupled to the functional section 20.

The lens section 10 has a curved surface shape that resembles a surface shape of the eyeball 100. The lens section 10 has, for example, a circular shape when viewed from front. The lens section 10 has a diameter having a value larger than a diameter of the pupil 110 when being enlarged in a dark environment. The lens section 10 may be a lens having an eyesight correction function intended to correct nearsightedness, farsightedness, astigmatism, etc., or may be a light-transmissive substrate not having such an eyesight correction function. The lens section 10 includes, for example, a light-transmissive resin, and serves as a supporting substrate that supports the functional section 20 and the terminal 30.

The functional section 20 is formed, for example, on an outer edge of the lens section 10. For example, as illustrated in FIG. 1, the functional section 20 is formed at a position not opposed to the pupil 110 when the contact lens 1 is worn on the eyeball 100. The functional section 20 may be formed at a position that does not affect light passing through the pupil 110 (for example, a position opposed to the iris 120).

The functional section 20 may be, for example, a sensor element that acquires biological information of a user wearing the contact lens 1 and outputs the biological information as an electrical signal. The sensor element is, for example, a device that detects, for example, specific ingredients contained in tears (for example, salt content, oxygen, lipid, blood glucose values, or hormonal substances). In this case, the electrical signal acquired by being detected by the sensor element includes information about the ingredients of the tears. The sensor element may be, for example, a device that detects a line of sight, a device that detects states of blood vessels inside an eyeball, a device that detects pulses of blood vessels inside an eyeball, a device that detects an eye pressure, or a device that detects opening/closing of an eyelid. The sensor element may acquire information other than the biological information. The sensor element may be, for example, a device that detects outside brightness, a device that detects vibrations, or a device that detects a temperature.

In a case where the sensor element includes a memory, the sensor element may store the acquired biological information in the memory without outputting the electrical signal. The biological information stored in the memory may be read from the memory, for example, when the contact lens 1 is removed from the eyeball 100 and is contained in a cradle.

For example, as illustrated in FIG. 1, the terminal 30 is formed on the outer edge of the lens section 10. For example, as illustrated in FIG. 2, the terminal 30 may be formed to protrude outside the outer edge of the lens section 10. The terminal 30 is disposed at a position close to an outer corner of an eye when the contact lens 1 is worn on the eyeball 100. Accordingly, when an external terminal 2B of an external device 2 to be described later is physically coupled to the terminal 30, it is possible to extend a line 2A from the contact lens 1 in a horizonal direction and draw the line 2A from the outer corner of the eye to outside. It is to be noted that the terminal 30 may be disposed at a position close to an inner corner of the eye when the contact lens 1 is worn on the eyeball 100. In this case, when the external terminal 2B of the external device 2 is physically coupled to the terminal 30, it is possible to extend the line 2A from the contact lens 1 in the horizontal direction and draw the line 2A from the inner corner of the eye to outside.

For example, as illustrated in FIGs. 1 and 2, the functional section 20 may be formed at a position not opposed to the pupil 110 when the contact lens 1 is worn on the eyeball 100. The reason for this is to prevent the functional section 20 from blocking a view. In a case where the functional section 20 is a sensor element, for example, the terminal 30 may output, to the external device 2 to be described later, electric power inputted from the external device 2 to be described later, or may output, to the external device 2 to be described later, an electrical signal outputted from the functional section 20.

The terminal 30 is provided on a surface of the lens section 10, and is configured to be physically couplable to the external terminal 2B of the external device 2 to be described later. The terminal 30 is configured to be couplable to the external terminal 2B by magnetic force, for example. For example, in a case where the external terminal 2B is a pad-shaped electrode including a ferromagnet, the terminal 30 is a pad-shaped electrode including a ferromagnet similarly to the external terminal 2B. The terminal 30 may be configured to be couplable to the external terminal 2B by mating, for example. In a case where the external terminal 2B includes a general-purpose socket or plug, the terminal 30 includes a plug or a socket mated with the external terminal 2B, for example.

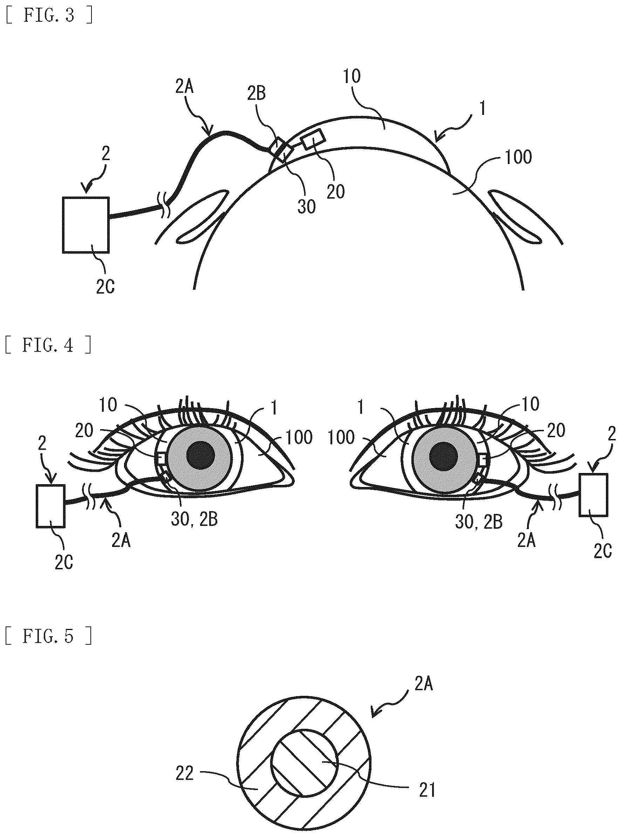

The external device 2 includes, for example, a main body section 2C, the external terminal 2B, and the line 2A that physically couples the main body section 2C and the external terminal 2B to each other, as illustrated in FIG. 3. The main body section 2C processes an electrical signal inputted into the contact lens 1 through the line 2A, for example. In addition, the main body section 2C supplies electric power to the contact lens 1 through the line 2A, for example. The external terminal 2B is physically coupled to the terminal 30 of the contact lens 1. The external terminal 2B is configured to be couplable to the terminal 30 by magnetic force, for example. In a case where the terminal 30 is a pad-shaped electrode including a ferromagnet, the external terminal 2B is, for example, a pad-shaped electrode including a ferromagnet similarly to the terminal 30. The external terminal 2B may be configured to be couplable to the terminal 30 by mating, for example. In a case where the terminal 30 includes a general-purpose socket or plug, the external terminal 2B includes a plug or a socket mated with the terminal 30, for example.

For example, as illustrated in FIG.4, it is assumed that a pair of contact lenses 1 are worn on both eyes. In this case, the terminal 30 of one contact lens 1 and the external terminal 2B of the external device 2 may be physically coupled to each other, and the terminal 30 of the other contact lens 1 and the external terminal 2B of another external device 2 may be physically coupled to each other.

FIG. 5 illustrates an example of a cross-sectional configuration of the line 2A. The line 2A is configured to enable, for example, transmission of an electrical signal from the functional section 20 to the main body section 2C and transmission of electric power from the main body section 2C to the functional section 20. The line 2A includes, for example, a conductive line 21 and a resin film 22 that covers the conductive line 21. The conductive line 21 includes, for example, a carbon nanotube, gold, silver, or copper. The resin film 22 includes, for example, a resin material that does not easily cause a discomfort feeing, a unpleasant feeing, or an allergic reaction when the line 2A comes into contact with the eyeball 100. The resin film 22 preferably includes a resin material that makes it possible to reduce friction at an edge of an eyelid, and preferably includes, for example, silicone, a fluorine resin, or the like.

The line 2A preferably has elasticity enough not to interfere with blinking. The elastic coefficient of the line 2A is preferably 0.7 or less. In addition, the thickness of the line 2A is preferably, for example, 10 nm (a typical thickness of a carbon nanotube) or more and 0.2 mm (a typical thickness of a head hair) or less.

### [Effects]

Next, description is given of effects of the contact lens 1 according to the present embodiment.

In a case where data is transmitted from a contact lens to peripheral equipment, wireless communication is typically used. In a case where the contact lens is worn on an eyeball, the area of the contact lens is small, and the contact lens gets wet with tears, which easily causes a decrease in communication sensitivity, and easily causes a slowdown in communication speed by noise superimposed on a signal. In addition, it is difficult to mount a battery having a large capacity on the contact lens, which limits functions implementable in the contact lens, and long-time use of a battery is not expected. It is possible to wirelessly supply electric power to the contact lens. However, it is difficult to dispose an electric power source in proximity to the contact lens, which limits suppliable electric power. As described above, in wireless communication, limitations are put on a communication band, a mounting function, used electric power, supplied electric power, a driving time, and the like. In addition, the contact lens is physically isolated from outside except for an eye; therefore, it is not possible to exchange an object between the contact lens and the outside. As described above, due to various limitations described above, it has been extremely difficult to provide a contact lens as a wearable device that is endurable for practical use.

Meanwhile, in the present embodiment, the functional section 20 provided in the lens section 10 is physically coupled to the terminal 30. The terminal 30 is configured to be physically couplable to the external terminal 2B. This makes it possible for the contact lens 1 to be physically coupled to the external device 2 including the external terminal 2B through the terminal 30. As a result, it is possible to exchange an object between the contact lens 1 and the external device 2 without various limitations caused in wireless communication. This makes it possible to reduce limitations on practically using the contact lens 1 and the pair of contact lenses 1 as a wearable device.

In addition, in the present embodiment, the terminal 30 is provided on the surface of the lens section 10, and is configured to be physically couplable to the external terminal 2B by mating or magnetic force, which makes it possible to easily couple the external terminal 2B to the terminal 30. This makes it possible to reduce limitations on practically using the contact lens 1 and the pair of contact lenses 1 as a wearable device.

In addition, in the present embodiment, electric power is supplied from the external device 2 to the functional section 20, and a signal corresponding to information acquired by the functional section 20 is outputted from the functional section 20 to the external device 2. This makes it possible for the external device 2 to make use of the information acquired by the functional section 20.

### <2. Modification Examples of First Embodiment>

Next, description is given of modification examples of the contact lens 1 according to the first embodiment described above.

### [Modification Example 1-1]

In the first embodiment described above, in a case where the external device 2 (the main body section 2C) is coupled to the terminal 30, the lens section 10 may be displaced from a predetermined position by contact of the line 2A, which physically couples the terminal 30 and the external device 2 (the main body section 2C) to each other, with an edge of an eyelid, or the like. Accordingly, the lens section 10 is preferably configured to be able to return to the predetermined position from a position where the lens section 10 is displaced.

For example, when the contact lens 1 is worn on an eye, at least one of a thickness distribution and a barycenter of the lens section 10 is preferably adjusted to locate the terminal 30 at a desired position in the eye. For example, an upper portion of the lens section 10 may be relatively thin, and a lower portion of the lens section 10 may be relatively thick. In addition, for example, the upper portion and the lower portion of the lens section 10 may be relatively thinner than a middle portion of the lens section 10. In addition, the barycenter of the lens section 10 may be located in the lower portion of the lens section 10.

In addition, for example, as illustrated in FIG. 6, the pair of contact lenses 1 may be provided with a strap-shaped coupling section 40 that physically couples the lens section 10 of one contact lens 1 and the lens section 10 of the other contact lens 1 to each other while maintaining a space corresponding to a pupil distance. The coupling section 40 includes, for example, a resin material that does not easily cause a discomfort feeing, a unpleasant feeing, or an allergic reaction when the coupling section 40 comes into contact with the eyeball 100. The coupling section 40 preferably includes a resin material that makes it possible to reduce friction at an edge of an eyelid, and preferably includes, for example, silicone, a fluorine resin, or the like.

### [Modification Example 1-2]

In the first embodiment described above, the contact lens 1 may include, for example, a plurality of terminals 30 as illustrated in FIG. 7. In this case, the plurality of terminals 30 may be provided for one functional section 20, or one of the terminals 30 may be provided for each functional section 20, for example, as illustrated in FIG. 7. For example, each of the terminals 30 is physically coupled to the external terminal 2B, and is further coupled to the line 2A through the external terminal 2B, as illustrated in FIG. 8.

### [Modification Example 1-3]

In the first embodiment described above, the functional section 20 may have a configuration that collects tears in an eye, or may be configured to supply a medicine, a liquid such as physiological saline solution, and a gas to the eyeball 100. In this case, it is necessary for the line 2A to have a configuration that is able to carry tears collected by the functional section 20 to the main body section 2C and carry a medicine, a liquid such as physiological saline solution, and a gas supplied from the main body section 2C to the functional section 20.

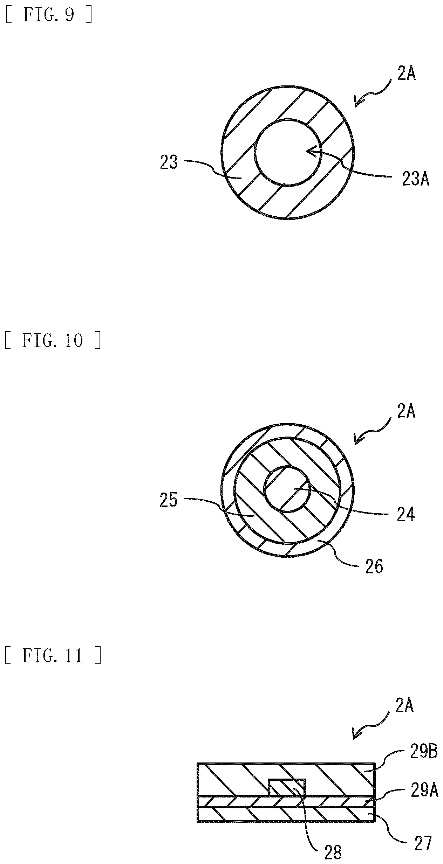

In the present modification example, the line 2A has a hollow structure, and may include a hollow line 23 having a cavity section 23A inside, for example, as illustrated in FIG. 9. The hollow line 23 includes, for example, a resin material that does not easily cause a discomfort feeing, a unpleasant feeing, or an allergic reaction when the hollow line 23 comes into contact with the eyeball 100. The hollow line 23 preferably includes a resin material that makes it possible to reduce friction at an edge of an eyelid, and preferably includes, for example, silicone, a fluorine resin, or the like.

### [Modification Example 1-4]

In the first embodiment described above, the functional section 20 may have, for example, a configuration that is able to irradiate a retina with light. The functional section 20 may have, for example, a configuration that is able to irradiate the retina with spot-like light, or a configuration that is able to irradiate the retina with image light of a plurality of pixels. The functional section 20 may include, for example, a prism or a diffraction element.

In addition, in the first embodiment described above, the functional section 20 may be, for example, an optical element that responds to light incident from the terminal 30. Examples of the optical element include a fluorescent element, a wavelength conversion element, and the like.

In a case where the functional section 20 is configured as described above, it is necessary for the line 2A to have, for example, a configuration that is able to transmit, to the functional section 20, light outputted from the main body section 2C. The line 2A may be, for example, an optical fiber as illustrated in FIG. 10. For example, as illustrated in FIG. 10, the line 2A includes a stick-shaped core layer 24 in a central portion, includes a cladding layer 25 that covers a periphery of the core layer 24, and further includes a coating layer 26 that protects the cladding layer 25. The core layer 24 includes a material having a higher refractive index than the refractive index of the cladding layer 25. The coating layer 26 includes, for example, a resin material that does not easily cause a discomfort feeing, a unpleasant feeing, or an allergic reaction when the coating layer 26 comes into contact with the eyeball 100. The coating layer 26 preferably includes a resin material that makes it possible to reduce friction at an edge of an eyelid, and preferably includes, for example, silicone, a fluorine resin, or the like.

The line 2A may be, for example, a light waveguide as illustrated in FIG. 11. The line 2A includes, on a substrate 27, for example, a stick-shaped core layer 28, and a lower cladding layer 29A and an upper cladding layer 29B between which the stick-shaped core layer 28 is vertically interposed, as illustrated in FIG. 11. The core layer 28 includes a material having a higher refractive index than the refractive indices of the lower cladding layer 29A and the upper cladding layer 29B.

### <3. Second Embodiment>

Next, description is given of a contact lens 3 according to a second embodiment of the present disclosure. It is to be noted that common components to those in the embodiment described above are denoted by same reference numerals.

FIG. 12 illustrates an example of a state in which the contact lens 3 is worn on the eyeball 100. The contact lens 3 performs wired communication with the external device 2. An upper portion and a lower portion of the eyeball 100 are covered with an upper eyelid and a lower eyelid, and an upper portion and a lower portion of the contact lens 1 worn on the eyeball 100 are also covered with the upper eyelid and the lower eyelid.

The contact lens 3 includes the lens section 10 worn on the eyeball 100, the functional section 20 provided in the lens section 10, a line 50 having one end physically coupled to the functional section 20, and the terminal 30 physically coupled to another end of the line 50.

The functional section 20 may be, for example, a sensor element that acquires biological information of a user wearing the contact lens 3 and outputs the biological information as an electrical signal. The sensor element is, for example, an element similar to the sensor element in the first embodiment described above.

For example, as illustrated in FIG. 12, the terminal 30 is physically coupled to the functional section 20 in the lens section 10 through the line 50. Accordingly, the line 50 is provided at a position apart from the lens section 10 and the eyeball 100. For example, as illustrated in FIG. 12, the functional section 20 is formed at a position not opposed to the pupil 110 when the contact lens 3 is worn on the eyeball 100. The functional section 20 may be formed at a position that does not affect light passing through the pupil 110 (for example, a position opposed to the iris 120). In a case where the functional section 20 is a sensor element, the terminal 30 may output, to the functional section 20, electric power inputted from the external device 2 or may output, to the external device 2, an electrical signal outputted from the functional section 20.

The terminal 30 is configured to be physically couplable to the external terminal 2B of the external device 2. The terminal 30 is configured to be couplable to the external terminal 2B by magnetic force, for example. For example, in a case where the external terminal 2B is a pad-shaped electrode including a ferromagnet, the terminal 30 is a pad-shaped electrode including a ferromagnet similarly to the external terminal 2B. The terminal 30 may be configured to be couplable to the external terminal 2B by mating, for example. In a case where the external terminal 2B includes a general-purpose socket or plug, the terminal 30 includes a plug or a socket mated with the external terminal 2B, for example.

The external device 2 includes, for example, the main body section 2C, the external terminal 2B, and the line 2A that physically couples the main body section 2C and the external terminal 2B to each other, as illustrated in FIG. 13. The main body section 2C processes an electrical signal inputted into the contact lens 3 through the line 2A, for example. In addition, the main body section 2C supplies electric power to the contact lens 3 through the line 2A, for example. The external terminal 2B is physically coupled to the terminal 30 of the contact lens 3. The external terminal 2B is configured to be couplable to the terminal 30 by magnetic force, for example. In a case where the terminal 30 is a pad-shaped electrode including a ferromagnet, the external terminal 2B is, for example, a pad-shaped electrode including a ferromagnet similarly to the terminal 30. The external terminal 2B may be configured to be couplable to the terminal 30 by mating, for example. In a case where the terminal 30 includes a general-purpose socket or plug, the external terminal 2B includes a plug or a socket mated with the terminal 30, for example.

For example, as illustrated in FIG.14, it is assumed that a pair of contact lenses 3 are worn on both eyes. In this case, the terminal 30 of one contact lens 3 and the external terminal 2B of the external device 2 may be physically coupled to each other, and the terminal 30 of the other contact lens 3 and the external terminal 2B of another external device 2 may be physically coupled to each other. In this case, both the external devices 2 have a communication section that enables wireless communication between both the main body sections 2C, and may synchronously control functions and information of both the contact lenses 3 through both the communication sections.

FIG. 15 illustrates an example of a cross-sectional configuration of the line 50. The line 50 is configured to enable, for example, transmission of an electrical signal from the functional section 20 to the main body section 2C and transmission of electric power from the main body section 2C to the functional section 20. The line 50 includes, for example, a conductive line 51 and a resin film 52 that covers the conductive line 51. The conductive line 51 includes a carbon nanotube, gold, silver, copper, or a mixture of at least two of these materials. The resin film 52 includes, for example, a resin material that does not easily cause a discomfort feeing, a unpleasant feeing, or an allergic reaction when the line 50 comes into contact with the eyeball 100. The resin film 52 preferably includes a resin material that makes it possible to reduce friction at an edge of an eyelid, and preferably includes, for example, silicone, a fluorine resin, or the like.

The line 50 preferably has elasticity enough not to interfere with blinking. The elastic coefficient of the line 50 is preferably 0.7 or less. In addition, the thickness of the line 50 is preferably, for example, 10 nm (a typical thickness of a carbon nanotube) or more and 0.2 mm (a typical thickness of a head hair) or less. In addition, for example, as illustrated in FIG. 13, the line 50 may be disposed along the eyeball 100 not to interfere with blinking. In this case, of the line 50, a portion in contact with the surface of the lens section 10 is parallel or substantially parallel to a surface on the eyeball 100 side of the lens section 10, for example.

Of the line 50, the portion in contact with the surface of the lens section 10 is disposed, for example, at a position close to an outer corner of an eye when the contact lens 3 is worn on the eyeball 100. Accordingly, when the external terminal 2B of the external device 2 is physically coupled to the terminal 30, it is possible to extend the line 50 in a horizontal direction from the contact lens 3 and draw the line 50 from the outer corner of the eye to outside. It is to be noted that of the line 50, the portion in contact with the surface of the lens section 10 may be disposed at a position close to an inner corner of the eye when the contact lens 3 is worn on the eyeball 100. In this case, when the external terminal 2B of the external device 2 is physically coupled to the terminal 30, it is possible to extend the line 50 in the horizontal direction from the contact lens 3 and draw the line 50 from the inner corner of the eye to outside.

### [Effects]

Next, description is given of effects of the contact lens 3 according to the present embodiment.

In the present embodiment, the terminal 30 is physically coupled to the functional section 20 provided in the lens section 10. More specifically, the terminal 30 is physically coupled to the functional section 20 through the line 50. The terminal 30 is configured to be physically couplable to the external terminal 2B. This makes it possible for the contact lens 3 to be physically coupled to the external device 2 including the external terminal 2B through the terminal 30. As a result, it is possible to exchange an object between the contact lens 3 and the external device 2 without various limitations caused in wireless communication. This makes it possible to reduce limitations on practically using the contact lens 3 and the pair of contact lenses 3 as a wearable device.

In addition, in the invention the line 50 includes the conductive line 51 including a carbon nanotube, gold, silver, or copper. This makes it possible to transmit, to the external device 2, an electrical signal from the contact lens 3 and transmit electric power from the external device 2 to the contact lens 3. As described above, in the present embodiment, an electrical signal is exchanged between the contact lens 3 and the external device 2 by wire. This eliminates various limitations caused in wireless communication.

In addition, in the present embodiment, the conductive line 51 is covered with the resin film 52. Accordingly, selecting the resin film 52 makes it possible not to easily cause a discomfort feeing, a unpleasant feeing, or an allergic reaction when the line 50 comes into contact with the eyeball 100.

In addition, in the present embodiment, the elastic coefficient of the line 50 is 0.7 or less, which makes it possible to provide the line 50 without interfering with blinking. This makes it possible to reduce limitations on practically using the contact lens 3 and the pair of contact lenses 3 as a wearable device.

In addition, in the present embodiment, the line 50 has a hollow structure. This makes it possible to carry, to the external device 2, a liquid and a gas from the contact lens 3 and carry a liquid from the external device 2 to the contact lens 3. As described above, in the present embodiment, it is possible to exchange an object between the contact lens 3 and the external device 2. This makes it possible to reduce limitations on practically using the contact lens 1 and the pair of contact lenses 1 as a wearable device.

In addition, in the present embodiment, the line 50 includes an optical fiber or a light waveguide. This makes it possible to transmit, to the external device 2, light from the contact lens 3. As described above, in the present embodiment, it is possible to exchange light between the contact lens 3 and the external device 2. This makes it possible to reduce limitations on practically using the contact lens 1 and the pair of contact lenses 1 as a wearable device.

### <4. Modification Examples of Second Embodiment>

Next, description is given of modification examples of the contact lens 3 according to the second embodiment described above.

### [Modification Example 2-1]

In the second embodiment described above, in a case where the external device 2 (the main body section 2C) is coupled to the terminal 30, the lens section 10 may be displaced from a predetermined position by contact of the line 50, which physically couples the terminal 30 and the functional section 20 to each other, with an edge of an eyelid, or the like. Accordingly, the lens section 10 is preferably configured to be able to return to the predetermined position from a position where the lens section 10 is displaced.

For example, when the contact lens 3 is worn on an eye, at least one of a thickness distribution and a barycenter of the lens section 10 is preferably adjusted to locate the functional section 20 at a desired position in the eye. For example, an upper portion of the lens section 10 may be relatively thin, and a lower portion of the lens section 10 may be relatively thick. In addition, for example, the upper portion and the lower portion of the lens section 10 may be relatively thinner than a middle portion of the lens section 10. In addition, the barycenter of the lens section 10 may be located in the lower portion of the lens section 10.

In addition, for example, as illustrated in FIG. 16, the pair of contact lenses 3 may be provided with the strap-shaped coupling section 40 that physically couples the lens section 10 of one contact lens 3 and the lens section 10 of the other contact lens 3 to each other while maintaining a space corresponding to a pupil distance. The coupling section 40 includes, for example, a resin material that does not easily cause a discomfort feeing, a unpleasant feeing, or an allergic reaction when the coupling section 40 comes into contact with the eyeball 100. The coupling section 40 preferably includes a resin material that makes it possible to reduce friction at an edge of an eyelid, and preferably includes, for example, silicone, a fluorine resin, or the like.

### [Modification Example 2-2]

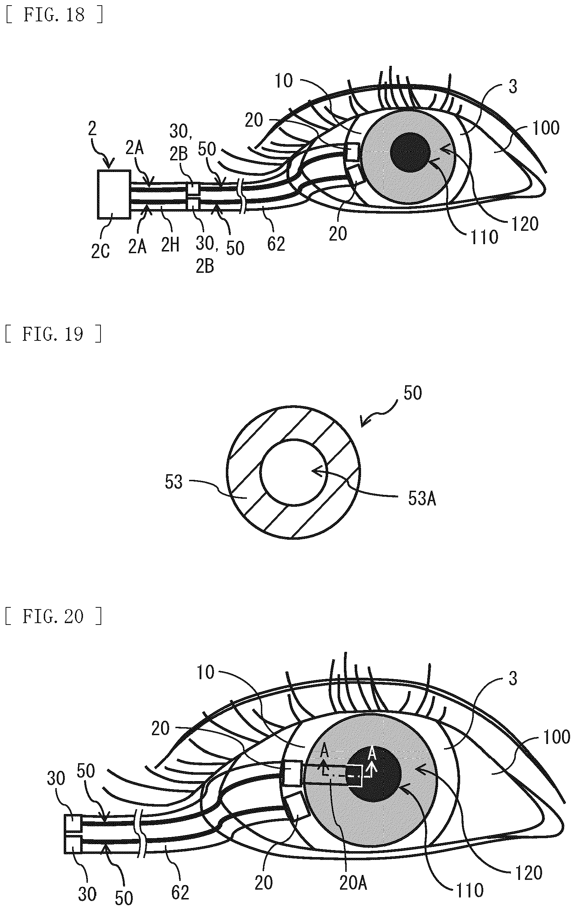

In the second embodiment described above and modification examples thereof, the contact lens 3 may include, for example, a plurality of terminals 30 as illustrated in FIG. 17. In this case, a plurality of lines 50 and a plurality of terminals 30 may be provided for one functional section 20, or one of the lines 50 and one of the terminals 30 may be provided for each functional section 20, for example, as illustrated in FIG. 17. For example, as illustrated in FIG. 18, each of the terminals 30 is physically coupled to the external terminal 2B, and is further coupled to the line 2A through the external terminal 2B. In this case, the contact lens 3 may further include a coating section 62 that bundles the plurality of lines 50. The external terminal 2B may include a plurality of lines 2A and further include a coating section 2H that bundles the plurality of line 2A. The coating section 62 and the coating section 2H include, for example, a resin material that does not easily cause a discomfort feeing, a unpleasant feeing, or an allergic reaction when the coating section 2H comes into contact with the eyeball 100. The coating section 2H preferably includes a resin material that makes it possible to reduce friction at an edge of an eyelid, and preferably includes, for example, silicone, a fluorine resin, or the like. In such a case, the external device 2 and the contact lens 3 are easily coupled to each other.

### [Modification Example 2-3]

In the second embodiment described above and the modification examples thereof, the functional section 20 may have a configuration that collects tears in an eye, or may be configured to supply a medicine, a liquid such as physiological saline solution, and a gas to the eyeball 100. In this case, it is necessary for the line 2A and the line 50 to have a configuration that is able to carry tears collected by the functional section 20 to the main body section 2C and carry a medicine, a liquid such as physiological saline solution, and a gas supplied from the main body section 2C to the functional section 20.

In the present modification example, the line 2A has a hollow structure, and may include the hollow line 23 having the cavity section 23A inside, for example, as illustrated in FIG. 9. The hollow line 23 includes, for example, a resin material that does not easily cause a discomfort feeing, a unpleasant feeing, or an allergic reaction when the hollow line 23 comes into contact with the eyeball 100. The hollow line 23 preferably includes a resin material that makes it possible to reduce friction at an edge of an eyelid, and preferably includes, for example, silicone, a fluorine resin, or the like.

In the present modification example, the line 50 also has a hollow structure, and may include a hollow line 53 having a cavity section 53A inside, for example, as illustrated in FIG. 19. The hollow line 53 includes, for example, a resin material that does not easily cause a discomfort feeing, a unpleasant feeing, or an allergic reaction when the hollow line 53 comes into contact with the eyeball 100. The hollow line 53 preferably includes a resin material that makes it possible to reduce friction at an edge of an eyelid, and preferably includes, for example, silicone, a fluorine resin, or the like.

### [Modification Example 2-4]

In the second embodiment described above and the modification examples 2-1 and 2-2, the functional section 20 may have, for example, a configuration that is able to irradiate a retina with light. The functional section 20 may have, for example, a configuration that is able to irradiate the retina with spot-like light, or a configuration that is able to irradiate the retina with image light of a plurality of pixels. The functional section 20 may include, for example, a prism, a mirror, or a diffraction element.

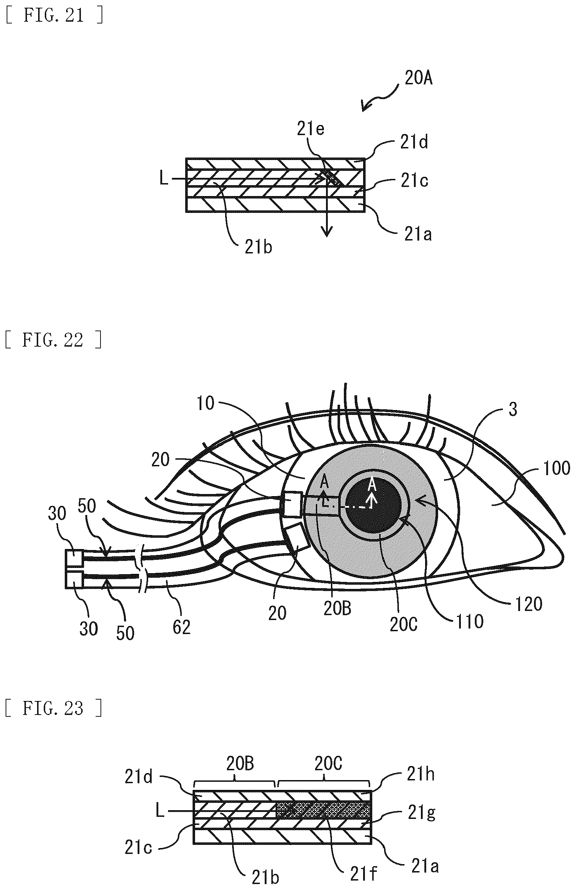

FIG. 20 illustrates an example of a state in which the contact lens 3 according to the present modification example is worn on an eye. FIG. 21 illustrates an example of a cross-sectional configuration taken along a line A-A of the contact lens 3 in FIG. 20. In the present modification example, at least one of one or a plurality of functional sections 20 provided in the contact lens 3 includes a light guiding section 20A extending from an end of the lens section 10 to the middle of the lens section 10. One end of the light guiding section 20A is provided, for example, in a middle portion of the contact lens. Th functional section 20 provided with the light guiding section 20A includes, for example, a light-emitting element that emits light L toward the light guiding section 20A. This light-emitting element operates (emits light) on the basis of a control signal inputted through the line 2A and the line 50. It is to be noted that in a case where the line 2A and the line 50 each include a light waveguide or an optical fiber as described later, it is possible to supply the light L from the external device 2; therefore, the light-emitting element described above may be omitted from the functional section 20.

The light guiding section 20A is a light waveguide, and includes, on a substrate 21a, for example, a strap-shaped core layer 21b, and a lower cladding layer 21c and an upper cladding layer 21d between which the strap-shaped core layer 21b is vertically interposed, as illustrated in FIG. 21. The core layer 21b includes a material having a higher refractive index than the refractive indices of the lower cladding layer 21c and the upper cladding layer 21d. The light guiding section 20A further includes, in the core layer 21b, for example, a mirror element 21e that reflects the light L having propagated through the core layer 21b toward a retina, as illustrated in FIG. 21. It is to be noted that the light guiding section 20A may include, in the substrate 21a, a diffraction element that controls the amount of diffusion of the light L reflected by the mirror element 21e. Providing the diffraction element makes it possible for a user to perceive that only a portion of a view shines.

FIG. 22 illustrates an example of a state in which the contact lens 3 according to the present modification example is worn on an eye. FIG. 23 illustrates an example of a cross-sectional configuration taken along a line A-A of the contact lens 3 in FIG. 22. In the present modification example, at least one of one or a plurality of functional sections 20 provided in the contact lens 3 includes, for example, a light guiding section 20B extending from an end of the lens section 10 to the middle of the lens section 10. One end of the light guiding section 20B is provided, for example, in a middle portion of the contact lens 3. The functional section 20 provided with the light guiding section 20B includes a light-emitting element that emits the light L toward the light guiding section 20B. The light-emitting element operates (emits light) on the basis of a control signal inputted through the line 2A and the line 50. It is to be noted that in a case where the line 2A and the line 50 each include a light waveguide or an optical fiber as descried later, it is possible to supply the light L from the external device 2; therefore, the light-emitting element described above may be omitted from the functional section 20.

The light guiding section 20B is a light waveguide, and includes, on the substrate 21a, for example, the strap-shaped core layer 21b, and the lower cladding layer 21c and the upper cladding layer 21d between which the strap-shaped core layer 21b is vertically interposed, as illustrated in FIG. 23. The core layer 21b includes a material having a higher refractive index than the refractive indices of the lower cladding layer 21c and the upper cladding layer 21d. It is to be noted that the mirror element 21e described above is not provided in the functional section 20, and the light L having propagated through the light guiding section 20B enters a light control section 20C. That is, the functional section 20 includes the light guiding section 20B and the light control section 20C. The light control section 20C is physically coupled to the light guiding section 20B, and is formed integrally with the light guiding section 20B. The light control section 20C is an element that responds to light incident through the light guiding section 20B.

The light control section 20C is provided in a middle portion of the lens section 10. The light control section 20C is a light waveguide similarly to the light guiding section 20B, and includes, on the substrate 21a, for example, a circular light control layer 21f that is provided in the same layer as the core layer 21b and is physically coupled to the core layer 21b, as illustrated in FIG. 23. The light control section 20C further includes, for example, a circular lower cladding layer 21g that is provided in the same layer as the lower cladding layer 21c and is physically coupled to the lower cladding layer 21c, and a circular upper cladding layer 21h that is provided in the same layer as the upper cladding layer 21d and is physically coupled to the upper cladding layer 21d, as illustrated in FIG. 23. The light control layer 21f includes a material having a higher refractive index than the refractive indices of the lower cladding layer 21g and the upper cladding layer 21h.

The light control layer 21f, the lower cladding layer 21g, and the upper cladding layer 21h may have a shape different from a circular shape. In addition, the light control layer 21f, the lower cladding layer 21g, and the upper cladding layer 21h may have a size enough to cover the pupil 110 when the contact lens 3 is worn on the eyeball 100, or may have a size enough to cover a portion of the pupil 110 when the contact lens 3 is worn on the eyeball 100. In addition, in a case where the lens section 10 is a stacked body, the lower cladding layers 21c and 21g, the upper cladding layers 21d and 21h, and the core layer 21b may be components of the stacked body of the lens section 10.

The light control layer 21f includes, for example, a material that performs color development, coloring, and light emission by absorbing the light L. Examples of the material that performs color development or coloring by absorbing the light L include photochromic materials (such as cyanine, phthalocyanine, azobenzene, and a diarylethene derivative). The light control layer 21f is caused to perform color development, coloring, or light emission to present a color in a view, which makes it possible to inform a user of change in a peripheral environment or a peripheral situation in an easy-to-understand manner. In addition, in a case where the light control layer 21f includes the material that performs color development or coloring by absorbing the light L, the light control layer 21f is caused to perform color development or coloring in the glare of outside light (such as sunlight and a light) to decrease light transmittance of the contact lens 3, which makes it possible to provide an effect like sunglasses to the contact lens 3.

In addition, in the present modification example, the light control section 20C may have a configuration other than the configuration described above, and may be, for example, an element that responds to light incident from the terminal 30, such as a fluorescent element or a wavelength conversion element.

In a case where the functional section 20 has a configuration as described above, it is necessary to configure the line 2A to enable, for example, transmission of light outputted from the main body section 2C to the line 50. The line 2A may be, for example, an optical fiber as illustrated in FIG. 10. For example, as illustrated in FIG. 10, the line 2A includes the stick-shaped core layer 24 in a central portion, includes the cladding layer 25 that covers a periphery of the core layer 24, and further includes the coating layer 26 that protects the cladding layer 25. The core layer 24 includes a material having a higher refractive index than the refractive index of the cladding layer 25. The coating layer 26 includes, for example, a resin material that does not easily cause a discomfort feeing, a unpleasant feeing, or an allergic reaction when the coating layer 26 comes into contact with the eyeball 100. The coating layer 26 preferably includes a resin material that makes it possible to reduce friction at an edge of an eyelid, and preferably includes, for example, silicone, a fluorine resin, or the like.

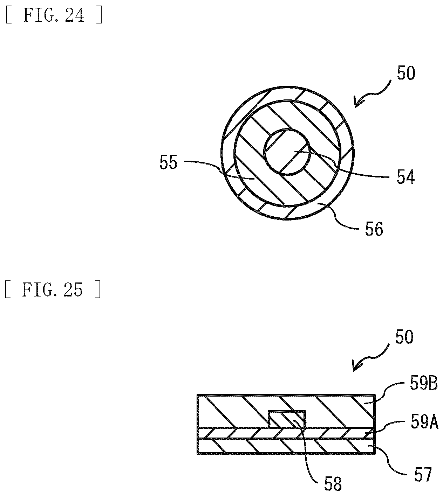

Further, it is also necessary to configure the line 50 to enable, for example, transmission of light having propagated through the line 2A to the functional section 20. The line 50 may be, for example, an optical fiber as illustrated in FIG. 24. For example, as illustrated in FIG. 24, the line 50 includes a stick-shaped core layer 54 in a central portion, includes a cladding layer 55 that covers a periphery of the core layer 54, and further includes a coating layer 56 that protects the cladding layer 55. The core layer 54 includes a material having a higher refractive index than the refractive index of the cladding layer 55. The coating layer 56 includes, for example, a resin material that does not easily cause a discomfort feeing, a unpleasant feeing, or an allergic reaction when the coating layer 56 comes into contact with the eyeball 100. The coating layer 56 preferably includes a resin material that makes it possible to reduce friction at an edge of an eyelid, and preferably includes, for example, silicone, a fluorine resin, or the like.

The line 2A may be, for example, a light waveguide as illustrated in FIG. 11. The line 2A includes, on the substrate 27, for example, the lower cladding layer 29A and the upper cladding layer 29B between which the stick-shaped core layer 28 is vertically interposed, as illustrated in FIG. 11. The core layer 24 includes a material having a higher refractive index than the refractive indices of the lower cladding layer 29A and the upper cladding layer 29B.

Further, the line 50 may be, for example, a light waveguide as illustrated in FIG. 25. The line 50 includes, on a substrate 57, for example, a lower cladding layer 59A and an upper cladding layer 59B between which a stick-shaped core layer 58 is vertically interposed, as illustrated in FIG. 25. The core layer 58 includes a material having a higher refractive index than the refractive indices of the lower cladding layer 59A and the upper cladding layer 59B.

### [Modification Example 2-5]

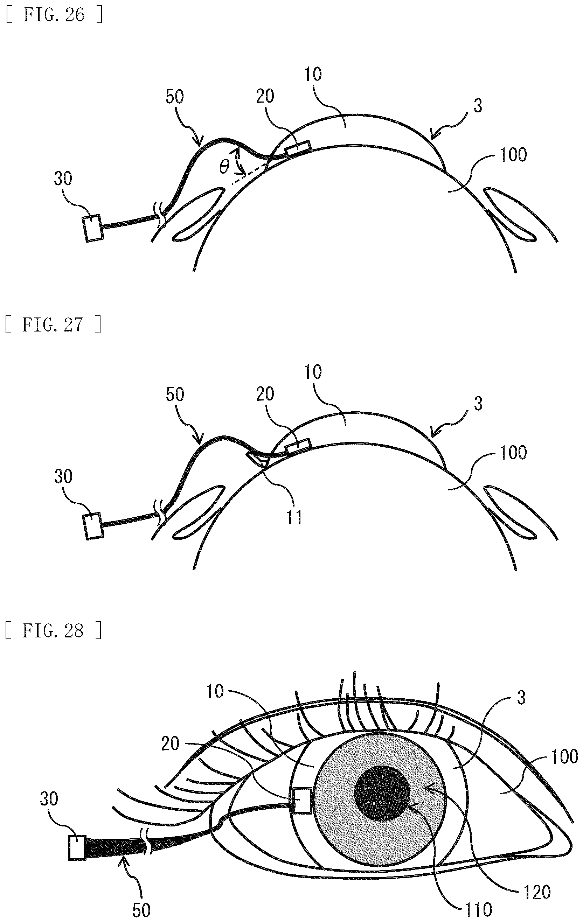

In the second embodiment described above and the modification examples thereof, for example, the line 50 may be disposed not in contact with the eyeball 100 as illustrated in FIG. 26. In this case, of the line 50, a portion in contact with the surface of the lens section 10 forms a predetermined angle θ with respect to a plane parallel to the surface on the eyeball 100 side of the lens section 10. The angle θ is, for example, an angle within a range of 0° to 90° both inclusive. This makes it possible to prevent the line 50 from constantly coming into contact with the surface of the eyeball 100.

In the present modification example, the contact lens 3 may include, for example, a protruding section 11 that protrudes from an outer edge of the lens section 10 on the outer edge of the lens section 10, as illustrated in FIG. 27. In this case, the protruding section 11 supports a portion in proximity to the surface of the lens section 10 in the line 50 to cause a portion in contact with the surface of the lens section 10 to form the predetermined angle θ with the plane parallel to the surface on the eyeball 100 side of the lens section 10. The protruding section 11 includes, for example, a resin having elasticity, and has flexibility enough to be bent by blinking. This makes it possible to surely prevent the line 50 from constantly coming into contact with the surface of the eyeball 100.

### [Modification Example 2-6]

According to the invention, the conductive line 51 is relatively thick in proximity to the terminal 30, as compared with in proximity to the functional section 20, for example, as illustrated in FIG. 28. This makes it possible to prevent the line 50 from interfering with blinking while reducing wiring resistance of the line 50.

### <5. Third Embodiment>

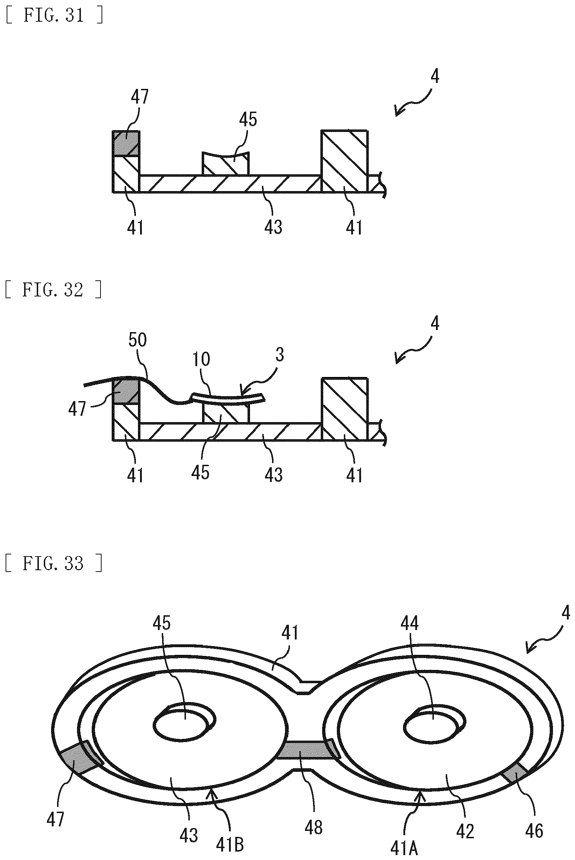

Next, description is given of a wearing device 4 according to a third embodiment of the present disclosure. FIG. 29 is a front view of an example of the wearing device 4. FIG. 30 is a back view of an example of the wearing device 4. FIG. 31 is a diagram illustrating an example of a cross-sectional configuration taken along a line A-A of FIG. 30.

The wearing device 4 is a device for wearing the pair of contact lenses 3 on both eyes. The wearing device 4 includes, for example, a lens holding section 44 that holds the lens section 10 provided in one contact lens 3, and a lens holding section 45 that holds the lens section 10 provided in the other contact lens 3. The lens holding section 44 corresponds to a specific example of a "lens holding section" and a "first lens holding section" in the present disclosure. The lens holding section 45 corresponds to a specific example of a "lens holding section" and a "second lens holding section" in the present disclosure. The lens holding section 44 holds the contact lens 3 that is worn on a right eye, for example. The lens holding section 45 holds the contact lens 3 that is worn on a left eye, for example.

The lens holding sections 44 and 45 each have, for example, a concave curved surface for easily holding the contact lens 3 as illustrated in FIG. 31, and hold the contact lens 3 by the curved surface, for example, as illustrated in FIG. 32. For example, surface tension by a preservative solution (for example, water) of the contact lens 3 makes it possible to develop a force of holding the contact lens 3 by the lens holding sections 44 and 45. It is to be noted that the force of holding the contact lens 3 may be achieved by giving low adhesiveness to the curved surfaces of the lens holding sections 44 and 45. In this case, the curved surfaces of the lens holding sections 44 and 45 include, for example, a silicone resin or the like. The lens holding sections 44 and 45 may include a flexible raw material (for example, a gel or the like) that has no influence even if the eyeball 100 touches the raw material.

The wearing device 4 further includes, for example, a line holding section 46 that holds one or a plurality of lines 50 of the contact lens 3 held by the lens holding section 44, and a line holding section 47 that holds one or a plurality of lines 50 of the contact lens 3 held by the lens holding section 45. The line holding section 46 corresponds to a specific example of a "line holding section" and a "first line holding section" in the present disclosure. The line holding section 47 corresponds to a specific example of a "line holding section" and a "second line holding section" in the present disclosure. The line holding sections 46 and 47 include, for example, a material having low adhesiveness.

The wearing device 4 further includes, for example, a lens section 42 that supports the lens holding section 44, a lens section 43 that supports the lens holding section 45, and a spectacle frame-shaped frame section 41 that supports the lens sections 42 and 43 and the line holding sections 46 and 47. The lens sections 42 and 43, the line holding sections 46 and 47, and the frame section 41 correspond to specific examples of a "supporting section" in the present disclosure. The lens section 42 corresponds to a specific example of a "lens section" and a "first lens section" in the present disclosure. The lens section 43 corresponds to a specific example of a "lens section" and a "second lens section" in the present disclosure. The frame section 41 corresponds to a specific example of a "frame section" in the present disclosure. The lens section 42 is provided in one opening 41A of the frame section 41. The lens section 43 is provided in another opening 41B of the frame section 41. The lens sections 42 and 43 are supported by the frame section 41, and each include, for example, a light-transmissive resin plate or glass pate. The line holding sections 46 and 47 are supported by the frame section 41, and are fixed at or in proximity to both end portions of the frame section 41, for example. The frame section 41 has a spectacle frame shape, and has a shape in which two frame sections having an elliptical ring shape or a long and thin polygonal ring shape are physically coupled to each other. The frame section 41 includes, for example, a resin material.

It is to be noted that in a case where the pair of contact lenses 3 to be worn with the wearing device 4 include the coupling section 40, the wearing device 4 may further include, for example, a line holding section 48 fixed in a portion of a space between two openings 41A and 41B of the frame section 41 as illustrated in FIG. 33. In this case, the line holding section 48 holds the coupling section 40. The line holding section 48 includes, for example, a material having low adhesiveness.

In the present embodiment, the lens section 10 is held by the lens holding section 44 or the lens holding section 45, and one or a plurality of lines 50 is held by the line holding section 46 or the line holding section 47. Accordingly, for example, holding the wearing device 4 up in front of eyes and pressing the wearing device 4 against a face makes it possible to wear the lens section 10 on an eye in a state in which the one or plurality of lines 50 is maintained at a desired position. Thus, in the present embodiment, using the wearing device 4 makes it possible to easily wear the contact lens 3 on the eye. This makes it possible to reduce limitations on practically using the contact lens 3 as a wearable device.

In the present embodiment, the line holding section 46 or the line holding section 47 is supported by the frame section 41 having an elliptical ring shape or a long and thin polygonal ring shape, and the lens holding section 44 or the lens holding section 45 is supported by the lens section 42 or the lens section 43. Accordingly, for example, holding the wearing device 4 up in front of eyes similarly to glasses and pressing the wearing device 4 against a face makes it possible to wear the lens section 10 on an eye in a state in which the one or plurality of lines 50 is maintained at a desired position. Thus, in the present embodiment, using the wearing device 4 makes it possible to easily wear the contact lens 3 on the eye. This makes it possible to reduce limitations on practically using the contact lens 3 as a wearable device.

In addition, in the present embodiment, two lens sections 10 are held by the lens holding section 44 and the lens holding section 45, and one or a plurality of lines 50 is held by the line holding section 46 and the line holding section 47. Accordingly, for example, holding the wearing device 4 up in front of eyes and pressing the wearing device 4 against a face makes it possible to wear the two lens sections 10 on both eyes in a state in which the one or plurality of lines 50 is maintained at a desired position. Thus, in the present embodiment, using the wearing device 4 makes it possible to easily wear the pair of contact lenses 3 on both eyes. This makes it possible to reduce limitations on practically using the pair of contact lenses 3 as a wearable device.

In the present embodiment, the line holding section 46 and the line holding section47 are supported by the spectacle frame-shaped frame section 41, and the lens holding section 44 and the lens holding section 45 are supported by the lens section 42 and the lens section 43. Accordingly, holding the wearing device 4 up in front of eyes similarly to glasses and pressing the wearing device 4 against a face makes it possible to wear the lens sections 10 on the eyes in a state in which the one or plurality of lines 50 is maintained at a desired position. Thus, in the present embodiment, using the wearing device 4 makes it possible to easily wear the contact lenses 3 on the eyes. This makes it possible to reduce limitations on practically using the contact lens 3 as a wearable device.

It is to be noted that in the present embodiment, the wearing device 4 may be a device for wearing one contact lens 3 on one eye. In this case, the wearing device 4 includes, for example, the lens holding section 45, the line holding section 47 that holds one or a plurality of lines 50 of the contact lens 3 held by the lens holding section 45, the lens section 43 that supports the lens holding section 45, and a ring-shaped frame section (for example, corresponding to a left-half portion of the frame section 41) that supports the lens section 43 and the line holding section 47. The lens section 43, the line holding section 47, and the frame section corresponds to specific examples of a "supporting section" in the present disclosure. The lens section 42 corresponds to a specific example of a "first lens section" in the present disclosure. The lens section 43 corresponds to a specific example of a "lens section" in the present disclosure. The frame section corresponds to a specific example of a "frame section" in the present disclosure. In such a case, for example, holding the wearing device 4 for a single eye up in front of one eye and pressing the wearing device 4 against a face makes it possible to wear one lens section 10 on the one eye in a state in which the one or plurality of lines 50 is maintained at a desired position. Further, it is possible to wear one lens section 10 on the other eye by a similar method. Thus, using the wearing device 4 for a single eye makes it possible to easily wear the pair of contact lenses 3 on both eyes. This makes it possible to reduce limitations on practically using the pair of contact lenses 3 as a wearable device.

It is to be noted that the effects described herein are merely exemplified. The effects of the present disclosure are not limited to the effects described herein. For example, in the third embodiment, the wearing device 4 has a configuration for both eyes, but may have, for example, a configuration for a single eye. The present disclosure may have effects other than the effects described herein.

For example, in the first embodiment described above, the external device 2 may further include, for example, an antenna line 2G that functions as an antenna (for example, a monopole type, a helical type, a sleeve type, or the like) as illustrated in FIG. 34. The antenna line 2G is disposed in parallel with the line 2A with a predetermined space interposed therebetween. In this case, the external device 2 includes a signal source 2F that is physically coupled to the antenna line 2G, and a wireless circuit 2E that controls the signal source 2F. Examples of a communication system in the wireless circuit 2E include LTE (Long Term Evolution), WLAN (Wireless Local Area Network), BT (Bluetooth (registered trademark)), LPWA (Low Power, Wide Area), and the like. The external device 2 may further include, for example, an operation section 2D that processes a signal acquired from the contact lens 1 through one or a plurality of lines 2A and outputs a thus-acquired signal. In this case, the external device 2 converts the signal outputted from the operation section 2D into a signal of a predetermined communication system in the wireless circuit 2E, and sends the signal as a radio wave from the antenna line 2G to outside. In such a case, the external device 2 is able to perform wireless communication with another electronic device through the antenna line 2G. As a result, for example, another electronic device is able to take charge of signal processing with a large load, and the external device 2 is able to take charge of necessary minimum signal processing only, which makes it possible to downsize the external device 2.

The external device 2 may include, for example, the coating section 2H that bundles the antenna line 2G together with one or a plurality of lines 2A as illustrated in FIG. 34. In such a case, coupling between the external device 2 and the contact lens 1 becomes easy. In addition, it is possible to make the length of the antenna line 2G equal to the length of the line 2A, which makes it possible to form the antenna line 2G with a long length, as compared with a case where the antenna line 2G is contained in the main body section 2C. As a result, it is possible to stabilize communication through the antenna line 2G.

The external device 2 may include, for example, a plurality of antenna lines 2G as illustrated in FIGs. 35 and 36. The plurality of antenna lines 2G is bundled together with one or a plurality of lines 2A by the coating section 2H, for example. Each of the antenna lines 2G is disposed in parallel to the line 2A with a predetermined space interposed therebetween. In this case, each of the antenna lines 2G may be directly coupled to the signal source 2F, for example, as illustrated in FIG. 35. In addition, the plurality of antenna lines 2G may be coupled to the signal source 2F through wiring in which the plurality of antenna lines 2G are coupled to each other in parallel, for example, as illustrated in FIG. 36.

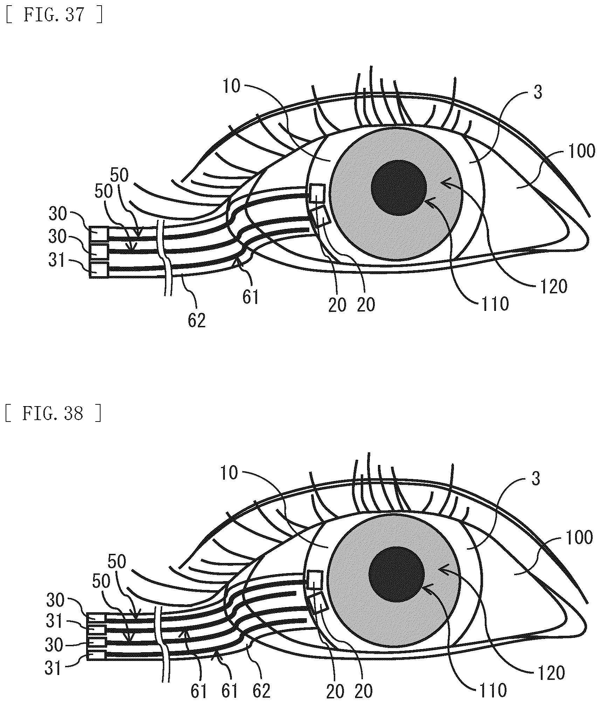

In addition, for example, in the second embodiment described above, the contact lens 3 may further include, for example, an antenna line 61 that functions as an antenna (for example, a monopole type, a helical type, a sleeve type, or the like), and a terminal 31 that is physically coupled to an end on a side opposite to the lens section 10 of the antenna line 62, as illustrated in FIG. 37. The antenna line 61 is disposed in parallel with the line 50 with a predetermined space interposed therebetween. The antenna line 61 is not coupled to the functional section 20 in the lens section 10, and is electrically separated from the functional section 20 in the lens section 10 in the contact lens 3.

The terminal 31 is configured to be physically couplable to an external terminal 2I (to be described later) of the external device 2. The terminal 31 is configured to be couplable to the external terminal 2I by magnetic force, for example. In a case where the external terminal 2I is a pad-shaped electrode including a ferromagnet, the terminal 31 is, for example, a pad-shaped electrode including a ferromagnet similarly to the external terminal 2I. The terminal 31 may be configured to be couplable to the external terminal 2I by mating, for example. In a case where the external terminal 2I includes a general-purpose socket or plug, the terminal 31 includes a plug or a socket mated with the external terminal 2I, for example.

In the second embodiment described above, the contact lens 3 may further include, for example, a plurality of antenna lines 61 and a plurality of terminals 31, each of which is physically coupled to an end on the side opposite to the lens section 10 of each of the antenna lines 61, as illustrated in FIG. 38. Each of the antenna lines 61 is disposed in parallel with the line 50 with a predetermined space interposed therebetween. Each of the antenna lines 61 is not coupled to the functional section 20 in the lens section 10, and is electrically separated from the functional section 20 in the lens section 10 in the contact lens 3.

In the second embodiment described above, the contact lens 3 may further include, for example, the coating section 62 that bundles one or a plurality of lines 50 and one or a plurality of antenna lines 61 as illustrated in FIGs. 37 and 38. In such a case, coupling between the external device 2 and the contact lens 3 becomes easy.

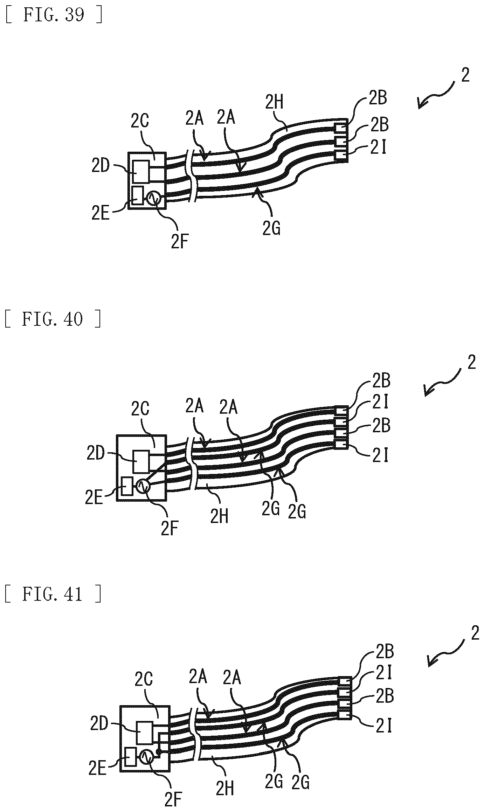

Here, in a case where the contact lens 3 is configured as illustrated in FIG. 37, the external device 2 may further include the external terminal 21 and the antenna line 2G that is physically coupled to the external terminal 2I as illustrated in FIG. 39. The external terminal 2I is configured to be physically couplable to the terminal 31 of the contact lens 3. The external terminal 2I is configured to be couplable to the terminal 31 by magnetic force, for example. In a case where the terminal 31 is a pad-shaped electrode including a ferromagnet, the external terminal 2I is, for example, a pad-shaped electrode including a ferromagnet similarly to the terminal 31. The external terminal 2I may be configured to be couplable to the terminal 31 by mating, for example. In a case where the terminal 31 includes a general-purpose socket or plug, the external terminal 2I includes a plug or a socket mated with the terminal 31, for example.

In a case where the contact lens 3 is configured as illustrated in FIG. 38, the external device 2 may further include, for example, a plurality of external terminals 2I, each of which is physically coupled to each of the antenna lines 2G, and a plurality of antenna line 2G, each of which is physically coupled to each of the external terminals 2I, as illustrated in FIGs. 40 and 41.

In a case where the contact lens 3 is configured as illustrated in FIGs. 37 and 38 and the external device 2 is configured as illustrated in FIGs. 39, 40, and 41, the antenna line 61 and the antenna line 2G are coupled to each other by coupling the contact lens 3 and the external device 2 to each other. In this case, for example, the external device 2 may further include, for example, the operation section 2D that processes a signal acquired from the contact lens 3 through one or a plurality of lines 2A and one or a plurality of lines 50 and outputs a thus-acquired signal. In this case, the external device 2 converts the signal outputted from the operation section 2D into a signal of a predetermined communication system in the wireless circuit 2E, and sends the signal as a radio wave from one or a plurality of antenna lines 2G and one or a plurality of antenna lines 61 to outside. In such a case, the external device 2 is able to perform wireless communication with another electronic device through the one or plurality of antenna lines 2G and the one or plurality of antenna lines 61. As a result, it is possible to increase the length of an antenna of the external device 2, as compared with a case where the antenna of the external device 2 includes only the antenna line 2G. As a result, it is possible to further stabilize communication through the antenna lines 2G and 61.

The external device 2 may include, for example, the coating section 2H that bundles the antenna line 2G together with one or a plurality of lines 2A, as illustrated in FIGs. 39, 40, and 41. In such a case, coupling between the external device 2 and the contact lens 1 becomes easy. In addition, it is possible to make the length of the antenna line 2G equal to the length of the line 2A, which makes it possible to form the antenna line 2G with a long length, as compared to a case where the antenna line 2G is contained in the main body section 2C. As a result, it is possible to stabilize communication through the antenna line 2F.

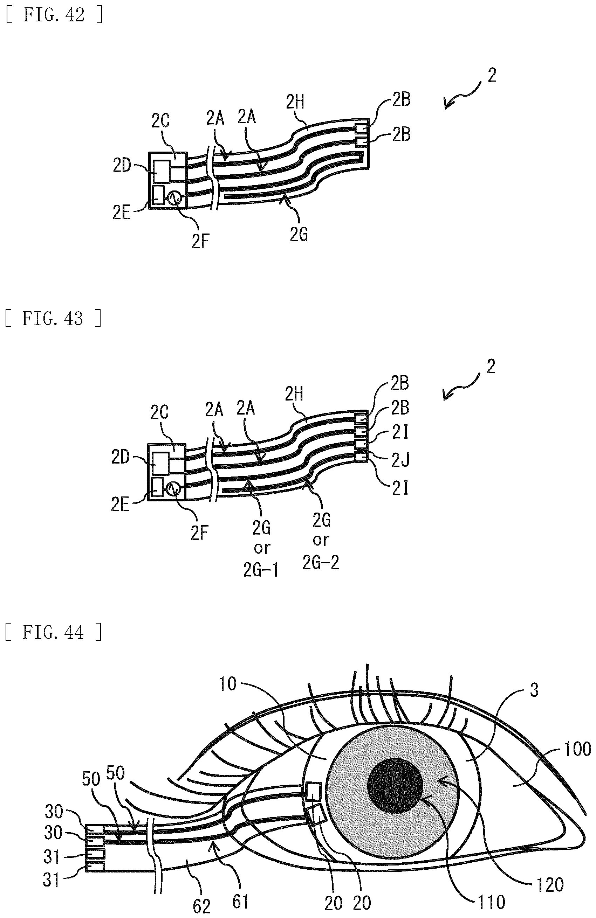

It is to be noted that in the external device 2, the antenna line 2G may be folded back, for example, as illustrated in FIG. 42. In such a case, it is possible to increase the length of the antenna of the external device 2 as much as the the antenna line 2G is folded back. As a result, it is possible to further stabilize communication through the antenna line 2G.

In addition, in the external device 2, two external terminals 2I may be coupled to each other by a wiring line 2J, for example, as illustrated in FIG. 43. In this case, the antenna line 2G (2G-1) coupled to one external terminal 2I may be directly coupled to the signal source 2F, and the antenna line 2G (2G-2) coupled to the other external terminal 2I may be electrically coupled to the signal source 2F through the two external terminals 2I and the antenna line 2G (2G-1). In such a case, it is possible to increase the length of the antenna of the external device 2 by the antenna line 2G (2G-2). As a result, it is possible to further stabilize communication through the antenna lines 2G.

It is to be noted that in a case where the antenna of the external device 2 is configured as illustrated in FIG. 43, in the contact lens 3, two terminals 31 coupled to two external terminals 2I may be, for example, dummy terminals that are not coupled to the antenna line 61 and the functional section 20, as illustrated in FIG. 44.

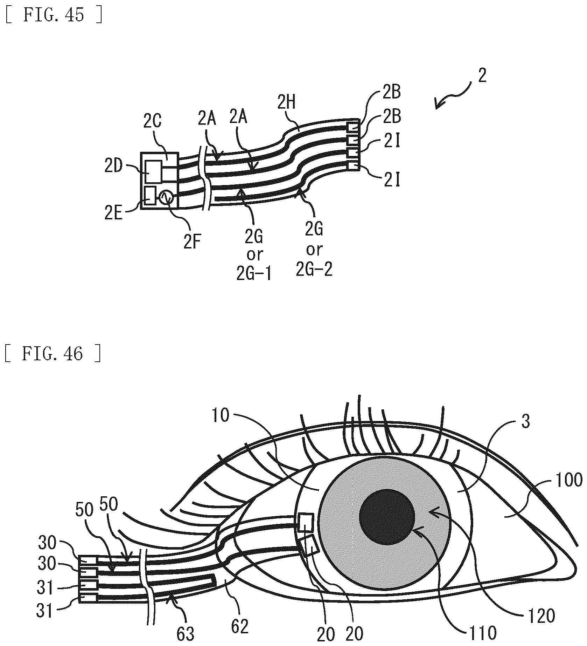

In addition, for example, as illustrated in FIG. 45, in the external device 2 illustrated in FIG. 43, the wiring line 2J that couples two external terminals 2I coupled to the antenna lines 2G to each other may be omitted. In this case, in the contact lens 3, two terminals 31 coupled to the two external terminals 2I may be coupled to each other by an antenna line 63, for example, as illustrated in FIG. 46. The antenna line 63 is disposed in parallel with the line 50, for example, and has a U-like shape extending in an extending direction of the line 50. In such a case, it is possible to increase the length of the antenna of the external device 2 by the antenna lines 63 and 2G-2. As a result, it is possible to further stabilize communication through the antenna lines 63 and 2G-2.

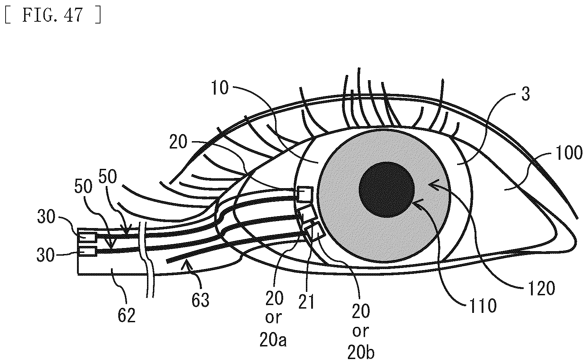

In addition, for example, as illustrated in FIG. 47, in a case where the conductive line 21 is provided that couples two functional sections 20 (20a and 20b) of a plurality of functional sections 20 to each other, one of the two functional sections 20 (20a and 20b) may include a signal source and a wireless circuit that controls the signal source. In this case, the antenna line 63 that is disposed in parallel with the line 50 with a predetermined space interposed therebetween may be provided in the functional section 20b. In such a case, it is possible to output a signal outputted from the functional section 20a to outside through the antenna line 63 even in a case where the external device 2 does not have a wireless function.

It is to be noted that the effects of the present disclosure are not necessarily limited to the effects described herein, and may be any of effects described in the present specification.

This application claims the benefits of Japanese Priority Patent Application JP2019-035150 filed with the Japan Patent Office on February 28, 2019.

It should be understood that those skilled in the art could conceive various modifications, combinations, sub-combinations, and alterations depending on design requirements and other factors, insofar as they are within the scope of the appended claims.

## Claims

1. A contact lens comprising:
a lens section that is worn on an eyeball;
one or a plurality of functional sections that is provided in the lens section; and
one or a plurality of terminals that is physically coupled to the one or plurality of functional sections, and is configured to be physically couplable to an external terminal; wherein
the contact lens further comprises one or a plurality of lines that has one end physically coupled to the one or plurality of functional sections, wherein
at least one of the one or plurality of terminals is physically coupled to another end of the one or plurality of lines;
at least one of the one or plurality of lines includes a conductive line including a carbon nanotube, gold, silver, copper, or a mixture of at least two of these materials; and
the conductive line is thicker in proximity to the terminal, as compared with in proximity to the functional section.

2. The contact lens according to claim 1, wherein at least one of the one or plurality of lines includes a resin film that covers the conductive line.

3. The contact lens according to claim 1, wherein the one or plurality of terminals is configured to be physically couplable to the external terminal by mating or magnetic force.

4. A pair of contact lenses comprising a contact lens according to claim 1.

## Patentansprüche

1. Kontaktlinse, umfassend:
einen Linsenabschnitt, der auf einem Augapfel getragen wird;
einen oder eine Vielzahl von Funktionsabschnitten, die in dem Linsenabschnitt bereitgestellt sind; und
einen oder eine Vielzahl von Anschlüssen, der/die mit dem einen oder der Vielzahl von Funktionsabschnitten physisch gekoppelt ist/sind und konfiguriert ist/sind, um mit einem externen Anschluss physisch gekoppelt zu werden; wobei
die Kontaktlinse ferner eine oder eine Vielzahl von Leitungen umfasst, deren eines Ende mit dem einen oder der Vielzahl von Funktionsabschnitten physisch gekoppelt ist, wobei
mindestens einer des einen oder der Vielzahl von Anschlüssen mit einem anderen Ende der einen oder der Vielzahl von Leitungen physisch gekoppelt ist;
mindestens eine der einen oder der Vielzahl von Leitungen eine leitfähige Leitung einschließt, die eine Kohlenstoffnanoröhre, Gold, Silber, Kupfer oder eine Mischung aus mindestens zwei dieser Materialien einschließt; und
die leitfähige Leitung in der Nähe des Anschlusses dicker ist als in der Nähe des Funktionsabschnitts.

2. Kontaktlinse nach Anspruch 1, wobei
wobei mindestens eine der einen oder der Vielzahl von Leitungen einen Harzfilm einschließt, der die leitfähige Leitung bedeckt.

3. Kontaktlinse nach Anspruch 1, wobei der eine oder die Vielzahl von Anschlüssen konfiguriert ist/sind, um durch Zusammenfügen oder Magnetkraft mit dem externen Anschluss physisch gekoppelt zu werden.

4. Paar Kontaktlinsen, umfassend eine Kontaktlinse nach Anspruch 1.

## Revendications

1. Lentille de contact comprenant :
une section de lentille portée sur un globe oculaire ;
une ou plusieurs sections fonctionnelles prévues dans la section de lentille ; et
un ou plusieurs terminaux physiquement accouplés à la ou aux sections fonctionnelles et configurés pour être physiquement accouplés à un terminal externe ; dans laquelle
la lentille de contact comprend en outre une ou plusieurs lignes dont une extrémité est physiquement accouplée à la ou les sections fonctionnelles, dans laquelle
au moins l'un parmi le ou les terminaux est physiquement accouplé à une autre extrémité de la ou des lignes ;
au moins l'une parmi la ou les lignes comporte une ligne conductrice comportant un nanotube de carbone, de l'or, de l'argent, du cuivre ou un mélange d'au moins deux parmi ces matériaux ; et
la ligne conductrice est plus épaisse à proximité du terminal qu'à proximité de la section fonctionnelle.

2. Lentille de contact selon la revendication 1,
dans laquelle au moins l'une parmi la ou les lignes comporte un film de résine qui recouvre la ligne conductrice.

3. Lentille de contact selon la revendication 1, dans laquelle le ou les terminaux sont configurés pour être physiquement accouplés au terminal externe par accouplement ou force magnétique.

4. Paire de lentilles de contact comprenant une lentille de contact selon la revendication 1.
